Europäisches Patentamt

European Patent Office

Office européen des brevets

(19)

(11) Numéro de publication: **0 217 690**
**A2**

(12) # DEMANDE DE BREVET EUROPEEN

(21) Numéro de dépôt: **86401806.4**

(22) Date de dépôt: **13.08.86**

(51) Int. Cl.⁴: **G 01 N 33/28**
**G 01 N 25/04**

(30) Priorité: **22.08.85 FR 8512611**

(43) Date de publication de la demande:
**08.04.87 Bulletin 87/15**

(84) Etats contractants désignés:
**AT DE GB NL SE**

(71) Demandeur: **ELF FRANCE, Société Anonyme dite:**
**Tour ELF 2 place de la Coupole La Défense 6**
**F-92400 Courbevoie(FR)**

(72) Inventeur: **Chague, Benoit**
**11 Avenue de Cadagne**
**F-69230 St. Genis Laval(FR)**

(72) Inventeur: **Esson, Serge**
**La Garenne**
**Luzinay F-38200 Vienne(FR)**

(72) Inventeur: **Julliat, Philippe**
**La Gaillardière**
**F-69440 Taluyers(FR)**

(74) Mandataire: **Kohn, Armand**
**5 Avenue Foch**
**F-92380 Garches(FR)**

(54) **Procédé et application pour la détermination des points de trouble et d'écoulement.**

(57) Procédé pour la détermination du point de trouble d'un liquide, qui consiste à refroidir le liquide progressivement, et à noter la température à laquelle apparaît un trouble ; les températures au coeur $(T_1)$ et à la périphérie $(T_2)$ du liquide sont measurées, on établit leurs courbes en fonction du temps $(\theta)$, et relève les changements de pente de ces courbes, le point de trouble étant la température $(T_1)$ au centre du liquide, qui correspond au changement de pente sur la courbe $T_2 = f'(\theta)$.

EP 0 217 690 A2

Croydon Printing Company Ltd

### Procédé et application pour la détermination des points de trouble et d'écoulement.

L'invention concerne un procédé perfectionné et un appareil pour la détermination du point de trouble et du point d'écoulement de différents liquides, en particulier des mélanges d'hydrocarbures que constituent les carburants, les huiles de graissage et les pétroles bruts. L'invention peut s'appliquer également à la détermination de la température de cristallisation commençante et à celle du point de congélation des liquides.

La connaissance de la température, à laquelle un liquide commence à se troubler du fait de l'apparition, en son sein, d'une phase solide, présente de l'importance dans certaines utilisations de ce liquide. Un cas typique est celui des carburants dont la circulation risque d'être entravée au-dessous de cette température, du fait de la formation de solides. Il en est de même pour une huile de graissage ou un liquide de transmission de chaleur. De même, et pour la même raison, il est important de connaître la température d'écoulement d'un liquide, c'est-à-dire celle à partir de laquelle le mouvement normal du liquide devient possible. Aussi existe-t-il des prescriptions officielles er des cahiers de charges imposant des normes précises aux températures en question, surtout dans le domaine des hydrocarbures, en ce qui concerne différents produits utilisés industriellement.

Ainsi, la détermination du point de trouble et de celui d'écoulement de produits pétroliers est-elle définie par la norme française T 60-105, en particulier pour les huiles lubrifiantes ou combustibles et les gazoles. Cette norme décrit également un appareil de dimensions et forme particulières pour l'application du mode opératoire normalisé. L'appareil comprend un bain réfrigérant, dans lequel plonge verticalement un tube à essai en verre ; ce dernier porte un bouchon percé au centre pour laisser passer un thermomètre qui, ainsi centré dans le tube, arrive jusqu'au fond de celui-ci. Le tube, contenant le liquide à examiner, est refroidi progressivement par le bain réfrigérant. La norme indique de retirer vivement le tube à essai mais sans remuer son contenu, chaque fois que la température lue descend de 1°C, pour vérifier la limpidité du liquide. On doit ensuite replacer le tube dans la jacquette réfrigérée, ces opérations manuelles ne devant pas durer plus de 3 secondes. Est considérée, comme "point de trouble", la température à laquelle un trouble distinct ou un brouillard apparaît dans le liquide au fond du tube.Avec le même appareil on détermine, selon la norme T 60-105, le point d'écoulement du liquide ; là l'observation sur le tube, rapidement retiré du bain, porte chaque fois sur le mouvement ou l'immobilité du liquide dans le tube que l'on incline. On considère que le point d'écoulement est atteint lorsque la surface du liquide ne se déforme plus pendant 5 secondes de maintien du tube en position horizontale. D'après la norme le point d'écoulement est la température lue plus 3°C. La naissance du capillaire du thermomètre est ici à 3 mm au-dessous de la surface du liquide.

Comme on le voit, les opérations normalisées comportent différents facteurs personnels pouvant agir défavorablement sur l'exactitude et la fidélité des résultats. L'appréciation visuelle du trouble laisse la place à des différences d'un opérateur à un autre. La rapidité des manipulations, leur temps limité à 3 secondes etc. peuvent également entraîner des écarts selon le tempérament des personnes qui les exécutent. Il en est de même de la lecture des températures simultanément avec le retrait du tube. Enfin, ces déterminations manuelles-visuelles prennent du temps et - dans un poste de contrôle fréquent - exigent la présence permanente d'un opérateur.

La présente invention a pour but le perfectionnement des déterminations décrites ci-dessus pour permettre leur réalisation rapide, précise, avec bonne répétabilité, peu coûteuse et affranchie de tout facteur personnel. L'invention permet, en effet, la détermination du point de trouble et de celui d'écoulement en parfait accord avec ce que donnent les opérations effectuées selon la norme T-60 105, mais avec les avantages susindiqués.

Le procédé suivant l'invention, pour la détermination du point de trouble d'un liquide, qui consiste à refroidir le liquide progressivement et à noter la température à laquelle apparaît un trouble, est caractérisé en ce que les températures au coeur ($T_1$) et à la périphérie ($T_2$) du liquide sont mesurées, on établit leurs courbes en fonction du temps ($\theta$), et l'on relève les changements de pente de ces courbes.

Conformément à l'invention, le point de trouble est la température ($T_1$) au centre du liquide, qui correspond au changement de pente sur la courbe $T_2 = \int'(\theta)$.

Le point d'écoulement est défini par la valeur de la température périphérale ($T_2$) au moment d'une inflexion sur la courbe $T_1 = \int'(\theta)$.

Les changements des pentes des courbes de température sont dûs aux effets thermiques se produisant lors de la formation d'un trouble, notamment du fait de la cristallisation des paraffines les plus lourds dans une huile ou gazole.

Le point d'écoulement des gazoles correspond au maximum de cet effet exothermique. Comme les moyens électroniques permettent à l'heure actuelle d'enregistrer et traiter aisément de telles informations, la présente invention peut être réalisée de façon simple et continue, sans les manipulations fastidieuses des méthodes normalisées, avec l'emploi d'un microprocesseur.

De plus, grâce à la double mesure de la température, au coeur du liquide et à la périphérie de celui-ci, l'invention rend possible la détermination simultanée des points de trouble et d'écoulement.

L'invention est illustrée non limitativement par la description, qui suit, d'un appareillage permettant de réaliser aisément le procédé décrit.

Fig. 1 représente schématiquement, en coupe axiale, l'éprouvette réfrigérée et ses accessoires, où ont lieu les mesures de température.

Fig. 2, également en coupe axiale schématique, montre un ensemble de 4 postes de détermination des points de trouble et d'écoulement.

Fig. 3 est le schéma d'un système électronique desservant le dispositif à éprouvette de la figure 1.

Fig. 4 comprend, à titre d'exemple, les courbes températures = $\int$ (temps) dans un cas particulier de détermination des points de trouble et d'écoulement par le procédé de l'invention.

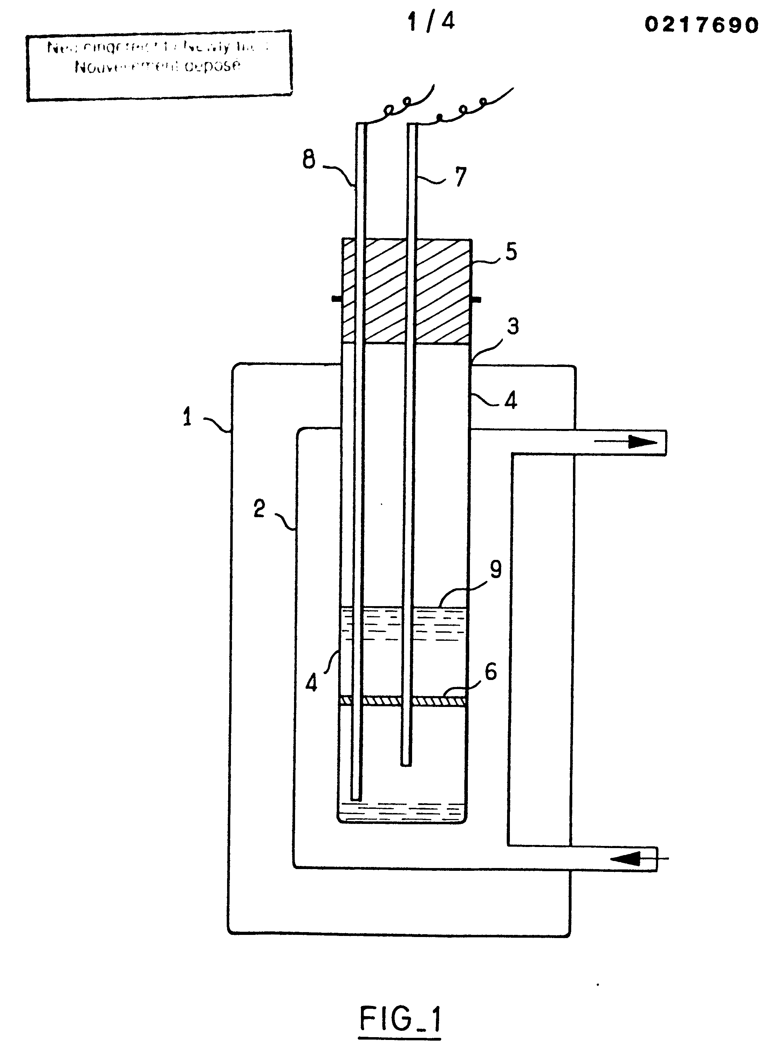

Sur la figure 1 on voit une enveloppe sous vide 1 entourant un réservoir 2, dans lequel on fait circuler en continu un fluide réfrigérant, comme indiqué par les flèches. Par une ouverture 3 dans le haut de l'enveloppe 1, une éprouvette 4 plonge dans le fluide réfrigérant.

L'éprouvette 4 est fermée par un bouchon 5 en polytétrafluoroéthylène que traversent les tiges de deux couples thermoélectriques ou sondes de températures Pt 100. Ces sondes traversent une entretoise 6, en matière plastique, notamment en polytétrafluoroéthylène. La partie active de la sonde 7 plonge dans la région centrale, inférieure, du liquide 9 dans l'éprouvette 4, tandis que celle de la sonde 8 se trouve près du fond et de la paroi extérieure de l'éprouvette.

Dans une forme d'exécution particulière, l'enveloppe 1 a un diamètre de 80 mm et une hauteur de 130 mm ; le diamètre du réservoir 2 est de 50 mm et la hauteur de 100mm ; l'éprouvette 4 présente un diamètre de 30 mm pour une hauteur de 120 mm, un trait indiquant le niveau du liquide se trouve à 50 mm au-dessus du fond.

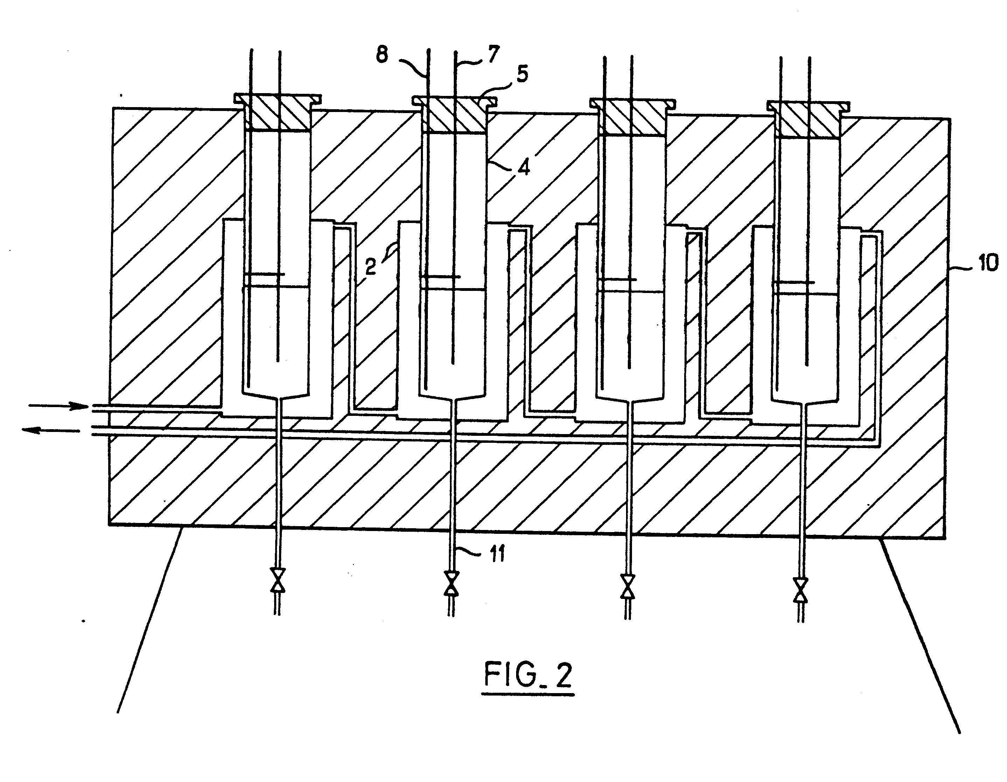

Fig. 2 représente un corps 10 rempli d'isolant thermique, dans lequel sont logés quatre réservoirs 2 de fluide réfrigérant, reliés en série. Dans chacun des réservoirs est plongé une éprouvette 4 semblable à celle de la figure 1, sauf qu'elle est munie d'une purge 11, permettant de vider l'éprouvette de son liquide, sans la retirer. On a ainsi un ensemble de 4 postes de travail permettant d'effectuer simultanément une série de 4 déterminations des points de trouble et d'écoulement.

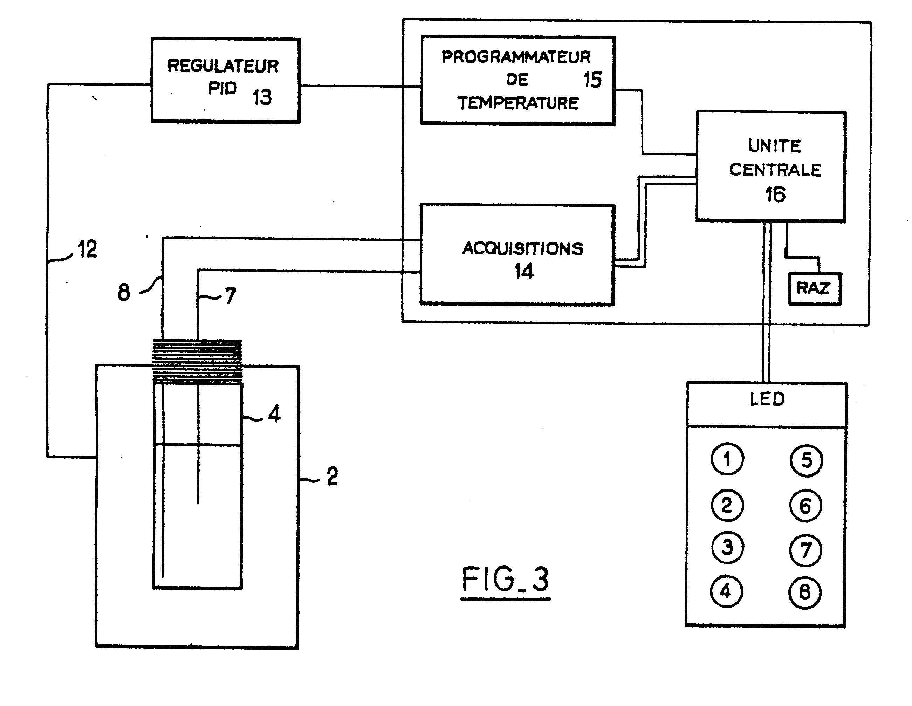

Sur la figure 3 on a indiqué sommairement les principales parties d'un microprocesseur relié à l'appareil de la fig.1. Les microprocesseurs étant bien connus, il n'y a pas lieu d'en faire une description détaillée. On notera seulement que le programmateur 1 pilote la consigne du régulateur 13 du bain.

Les signaux électriques des sondes de température 7 et 8 sont reçus dans le circuit d'acquisitions 14, pour être traités dans l'unité centrale 16 à laquelle est également relié le programmateur de température 15.

Le mode opératoire de l'utilisation de l'appareil suivant l'invention, dans le cas où l'on fait fonctionner les 4 postes de la figure 2, comprend les manipulations suivantes. On préchauffe le liquide à étudier à une température de 20° ou 25°C au-dessus du point de trouble présumé, et on le verse ensuite dans l'éprouvette jusqu'au trait de jauge prévu. L'éprouvette 4 est alors introduite dans le bain cryogénique 2 et l'on place les sondes 7 et 8 comme indiqué sur les figures 1 et 2.

Le système à microprocesseur est lancé par pression sur une touche appropriée,ce qui fait exécuter à l'appareil les fonctions suivantes : commande de la température du bain réfrigérant, test de la sonde 7 du 1er poste, acquisition des températures sur les quatre postes, traitement pour la détection des points de trouble et d'écoulement.

Des touches du microprocesseur ( 1 à 4 sur fig. 3) permettent la sélection de la sonde 7, de la sonde 8, des points de trouble et d'écoulement, et celle du point de cristallisation commençante, ainsi que le départ d'analyse.

D'autres touches (5 à 8 fig. 3) permettent la sélection respective de chacun des 4 postes de l'appareillage. Dès qu'un point est détecté il est affiché. Lorsque tous les points de trouble et d'écoulement sont détectés, on peut les lire à partir des touches 2, 5, 6, 7, 8 de la figure 3.

Une touche RAZ sert à relancer une manipulation.

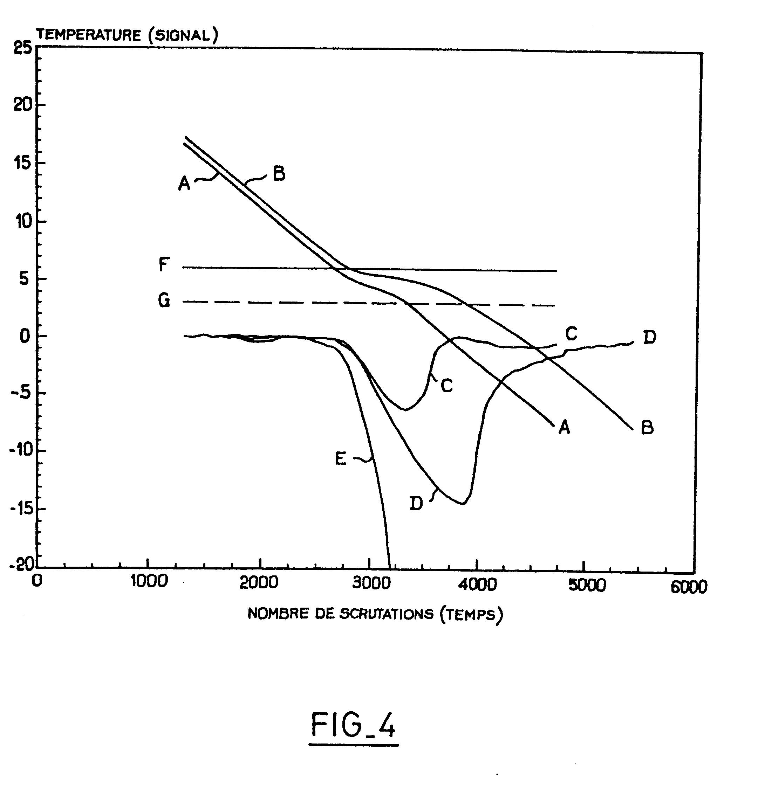

Le diagramme de la fig. 4 comporte une série de courbes se rapportant à la détermination simultanée du point de trouble et du point d'écoulement d'un gazole, prise à titre d'exemple, à l'aide de l'appareil automatique décrit plus haut.

Les abscisses portent les nombres de scrutations effectuées dans le liquide de l'éprouvette 4, pendant le refroidissement constant de 1°C par minute. Les scrutations étant effectuées à des intervalles de temps égaux, leur nombre équivaut à des durées déterminées. En ordonnées sont portées les températures acquises par les sondes thermométriques 7 et 8.

La ligne A représente les variations de la température $T_2$ indiquée par la sonde 8, voisine de la paroi de l'éprouvette 4, en fonction du temps, soit $T_2 = f(\theta)$. Le changement très net de la pente de cette courbe A, vers 2850 scrutations, détecte la cristallisation d'un composant du milieu. La température correspondante, c'est-à-dire le point de trouble, se lit sur la courbe B, pour la même abscisse. Cette indication cadre avec la ligne horizontale F qui correspond au point de trouble du même gazole, déterminé conformément aux normes.

La courbe B montre les variations de la température $T_1$, acquise à partir de la sonde centrale 7, en fonction du temps, soit $T_1 = f'(\theta)$. Son changement marqué de pente, vers 3200 scrutations, correspond au point d'écoulement $T_2$ qui se lit sur la sonde 8 au même temps (même nombre de scrutations), c'est-à-dire pour la même abscisse. Comparée avec l'ordonnée de la ligne G, cette température $T_2$ est en accord avec la valeur du point d'écoulement trouvé selon les normes (G).

Suivant un trait préféré de l'invention, afin de mieux saisir les inflexions des courbes du type A et B, on trace également les dérivées de celles-ci, soit les courbes comme C et D. La première courbure, sur chacune de ces lignes dérivées C et D, indique l'apparition d'une phase solide. Ainsi la première courbure (à gauche), bien plus nette que sur les courbes A et B, c'est-à-dire le premier changement de

0217690

pente sur C, détecte le point de trouble, dont la valeur est donnée par une courbe du type B correspondante, à la même abscisse. De façon analogue, le point d'écoulement est saisi avec plus de netteté par la courbe dérivée D et évalué sur A. A noter que, dans l'exemple illustré, les courbes C et D représentent les dérivées des courbes A et B filtrées et affectées d'un décalage d'abscisse.

L'invention comprend également, de préférence, le tracé d'une courbe cumulée E, afin d'éviter la détection de changements et phénomènes parasites qui ne sont pas dûs réellement à des solidifications survenant au sein du liquide étudié. Cette courbe E constitue le cumul de la courbe C avec remise à zéro chaque fois que cette dernière subit des écarts compris entre certaines limites prédéterminées. Ainsi, la détection du point de trouble n'est validée que si l'ordonnée de la courbe E dépasse un certain seuil préétabli.

La détection du point d'écoulement se fait après la détermination du point de trouble.

**REVENDICATIONS**

1. Procédé pour la détermination du point de trouble d'un liquide, qui consiste à refroidir le liquide progressivement, et à noter la température à laquelle apparaît un trouble, caractérisé en ce que les températures au coeur ($T_1$) et à la périphérie ($T_2$) du liquide sont mesurées, qu'on établit leurs courbes en fonction du temps ($\theta$), et que l'on relève les changements de pente de ces courbes, le point de trouble étant la température ($T_1$) au centre du liquide, qui correspond au changement de pente sur la courbe $T_2 = f(\theta)$.

2. Procédé suivant la revendication 1, caractérisée en ce que l'on détermine simultanément le point d'écoulement, en relevant la température ($T_2$) périphérale, lue au moment où un changement de pente apparaît sur la courbe $T_1 = f'(\theta)$.

3. Procédé suivant la revendication 1 ou 2, caractérisé en ce que l'on détermine les courbes dérivées des courbes $T_1 = f'(\theta)$ et $T_2 = f(\theta)$ et le premier changement significatif de pente de la courbe $T_2 = f(\theta)$ sert de détection du point de trouble, tandis que la première inflexion significative sur la courbe $T_1 = f''(\theta)$ sert de détection du point d'écoulement.

4. Procédé suivant la revendication 3, caractérisé en ce qu'une courbe cumulée (E) de la courbe dérivée (C) de $T_1 = f'(\theta)$ est établie avec remise à zéro chaque fois que cette courbe dérivée subit un écart inférieur à un seuil prédéterminé, la détection du point de trouble étant validée lorsque l'écart dépasse ce seuil.

5. Appareil pour la détermination du point de trouble et du point d'écoulement d'un liquide, qui comprend une éprouvette (4) pour contenir ce liquide (9), des moyens de refroidissement progressifs (2), ainsi que des moyens pour la mesure de la température du liquide (8), caractérisé en ce qu'il est muni de deux sondes thermométriques à couple thermoélectrique (7,8) dont la partie active de l'une (7) se trouve au centre du liquide, celle de la seconde (8) étant placée près de la paroi de l'éprouvette et du fond de celle-ci.

6. Appareil suivant la revendication 5, caractérisé en ce que deux sondes thermométriques (7,8) et les moyens de refroidissement (2) sont reliés à un système électronique (16) pour l'enregistrement de courbes de température en fonction du temps, à partir des deux sondes (7,8).

7. Appareil suivant la revendication 6, caractérisé en ce qu'il est muni d'éléments électroniques en vue de la comparaison des changements de pente de chacune des deux courbes avec l'autre courbe.

FIG_1

0217690

FIG. 2

REGULATEUR PID 13

PROGRAMMATEUR DE TEMPERATURE 15

UNITE CENTRALE 16

ACQUISITIONS 14

RAZ

12

8

7

4

2

LED

① 1 ⑤ 5

② 2 ⑥ 6

③ 3 ⑦ 7

④ 4 ⑧ 8

FIG_3

0217690

0217690

FIG_4